# EUROPÄISCHE PATENTSCHRIFT

(11) **EP 0 995 981 B1**
(45) Veröffentlichungstag und Bekanntmachung des Hinweises auf die Patenterteilung: **09.08.2006**
(21) Anmeldenummer: 99119657.7
(22) Anmeldetag: 04.10.1999
(51) Int. Cl.: G01N 11/04, A61F 2/46

(54) **Verfahren und Vorrichtung zur Bestimmung der Viskosität eines Knochenzements**
Process and apparatus for determining the viscosity of a bone cement
Procédé et dispositif pour déterminer la viscosité d'un ciment pour os

(30) Priorität: 21.10.1998 DE 19848477
(43) Veröffentlichungstag der Anmeldung: 26.04.2000
(73) Patentinhaber: Aesculap AG & Co. KG, 78532 Tuttlingen (DE)
(72) Erfinder: Grupp, Thomas, DE-73072 Donzdorf, Ortsteil Reichenbach (DE)
(74) Vertreter: Hoeger, Stellrecht & Partner Patentanwälte

(56) Entgegenhaltungen:
- US-A- 4 517 830
- US-A- 4 680 958
- US-A- 4 875 363

## Beschreibung

Die Erfindung betrifft ein Verfahren zur Bestimmung der Viskosität eines fließfähigen Knochenzements. Außerdem betrifft die Erfindung eine Vorrichtung zur Durchführung dieses Verfahrens.

Es ist bekannt, Prothesen in Hohlräumen von Knochen dadurch festzulegen, daß der Zwischenraum zwischen den Prothesen und der Innenwand des Hohlraums mit fließfähigem Knochenzement ausgefüllt wird. Dieser Knochenzement härtet nach dem Einbringen aus, und es ist wesentlich, daß er zu einem Zeitpunkt in den Zwischenraum eingefüllt wird, in dem seine Viskosität unter einem bestimmten Maximalwert liegt.

Um dies festzustellen, ist es beispielsweise bekannt, auf optischem Wege durch Bestrahlung der Oberfläche des Knochenzements dessen Viskosität zu bestimmen (DE 43 30 061 C1). Dieses Verfahren ist aufwendig und erfordert eine relativ komplizierte Apparatur. Eine weitere Apparatus zur Bestimmung der Viskosität über ein Kraftmessverfahren wir in US 4 680 958 beschrieben.

Es ist Aufgabe der Erfindung, ein Verfahren anzugeben, mit dem ein Benutzer in einfachster Weise feststellen kann, ob der zum Einfüllen vorgesehene Knochenzement die maximal zulässige Viskosität bereits überschritten hat oder nicht.

Diese Aufgabe wird bei einem Verfahren der eingangs beschriebenen Art erfindungsgemäß dadurch gelöst, daß man den fließfähigen Knochenzement mit einer Austragvorrichtung aus einem Vorratsbehälter durch eine Prüfleitung mit einer begrenzten Austragskraft hindurchfördert und beobachtet, wie weit der Knochenzement aus dem Vorratsbehälter in die Prüfleitung gedrückt wird.

Dadurch, daß die Austragvorrichtung nur mit einer begrenzten Austragkraft arbeitet, erfolgt ein Vorschieben des Knochenzements nur dann, wenn der Widerstand, den der Knochenzement dieser Förderung entgegensetzt, ein bestimmten Maß nicht überschreitet, wenn also die Viskosität unterhalb eines bestimmten Maximalwerts liegt. Wird die Viskosität größer, ist der Widerstand größer, den der Knochenzement der Austragvorrichtung entgegensetzt, und dann wird die begrenzte Austragskraft überschritten, die Austragvorrichtung unterbricht die Förderung, und dies führt dazu, daß der Knochenzement in der Prüfleitung nicht weiter vorgeschoben wird. Man kann dann sehr einfach beobachten, daß eine Förderung des Knochenzements in die Prüfleitung hinein nicht erfolgt, und dies ist ein Maß dafür, daß die Viskosität des Knochenzements einen bestimmten Maximalwert überschritten hat.

Dabei ist es vorteilhaft, wenn man den Knochenzement bis zum Erreichen einer maximalen Austragskraft mit konstantem Volumenstrom in die Prüfleitung fördert, dies läßt sich zum Beispiel dadurch erreichen, daß die Austragvorrichtung entsprechend gesteuert wird.

Es ist weiterhin Aufgabe der Erfindung, eine Vorrichtung zur Bestimmung der Viskosität eines fließfähigen Knochenzements mit einem Vorratsbehälter für den fließfähigen Knochenzement, einer aus diesem austretenden Austragleitung und einer Fördereinrichtung, die den fließfähigen Knochenzement aus dem Vorratsbehälter in die Austragleitung fördert, so auszugestalten, daß der Benutzer mit dieser Vorrichtung überprüfen kann, ob der Knochenzement den maximal zulässigen Viskositätswert überschritten hat.

Diese Aufgabe wird bei einer Vorrichtung der gattungsgemäßen Art erfindungsgemäß dadurch gelöst, daß der Vorratsbehälter mit einer Prüfleitung verbunden ist, daß der Fördereinrichtung ein Schaltelement zugeordnet ist, welches die Fördereinrichtung abschaltet, sobald ein Maximalwert einer Förderkraft überschritten wird, und daß die Prüfleitung derart beschaffen ist, daß feststellbar ist, wie weit Knochenzement in die Prüfleitung eingetreten ist.

Liegt die Viskosität des Knochenzements unterhalb des Maximalwerts, wird die Fördereinrichtung den Maximalwert der Förderkraft nicht erreichen, so daß Knochenzement durch die Prüfleitung gefördert wird. Dies ist für den Benutzer ohne weiteres feststellbar und ein Zeichen dafür, daß der Knochenzement noch im Verarbeitungsbereich liegt.

Wächst die Viskosität dagegen an, wird der Fließwiderstand des Knochenzements so groß, daß schließlich der Maximalwert der Förderkraft erreicht wird, die Fördereinrichtung schaltet ab, Knochenzement wird in der Prüfleitung nicht weiter gefördert, und auch dies ist für den Benutzer ohne weiteres erkennbar und ein Zeichen dafür, daß dieser Knochenzement nicht mehr eingefüllt werden kann, da seine Viskosität bereits zu hoch ist.

Das Schaltelement kann beispielsweise eine Kupplung sein.

Günstig ist es, wenn die Prüfleitung durchsichtig ist. Der Benutzer kann dann direkt beobachten, wie weit der Knochenzement in der Prüfleitung vorgeschoben wird.

Es ist weiterhin vorteilhaft, wenn die Prüfleitung im Abstand zum Vorratsbehälter eine Markierung trägt, die ein Maß dafür darstellt, wie weit Knochenzement mindestens in die Prüfleitung eintreten muß, wenn er noch verarbeitbar ist.

Es ist grundsätzlich möglich, daß die Prüfleitung durch die Austragleitung selbst gebildet wird, wenn die Viskosität so hoch wird, daß kein weiteres Vorschieben des Knochenzements mehr erfolgt, muß das Ausbringen des Knochenzements unterbleiben, die Austragleitung wird dann als Wegwerfteil ausgebildet.

Es ist auch möglich, daß aus dem Vorratsbehälter eine Prüfleitung und eine Austragleitung austreten, daß also zwei Leitungen austreten, von denen eine nur zur Prüfung der Viskosität verwendet wird, die andere nur zum Ausbringen des Knochenzements in den Zwischenraum zwischen Prothese und Knochenwand.

Es ist dann vorteilhaft, wenn die Austragleitung und die Prüfleitung verschließbar sind, so daß entweder die Prüfleitung oder die Austragleitung mit Knochenzement beschickt wird.

Es kann auch vorgesehen sein, daß die Prüfleitung abbrechbar ausgebildet ist, in diesem Falle ist weiterhin möglich, daß über die abgebrochene Prüfleitung die Austragleitung auf den Vorratsbehälter aufsetzbar ist.

Es ist dabei besonders vorteilhaft, wenn sich die Prüfleitung an die Austragleitung anschließt. Dies ermöglicht dem Benutzer, die Viskositätsprüfung durchzuführen und anschließend den vorderen Teil der Baueinheit aus Austragleitung und Prüfleitung abzubrechen, der verbleibende Teil, der die Austragleitung bildet, kann dann sofort zum Einfüllen des Knochenzementes in den Knochenhohlraum verwendet werden.

Bei einer anderen bevorzugten Ausführungsform ist vorgesehen, daß am Vorratsbehälter austauschbare Anschlußteile befestigbar sind, die wahlweise mit der Austragleitung oder mit einer Prüfleitung verbunden sind. Nach der Prüfung der Viskosität werden diese Anschlußteile ausgetauscht, so daß dann ein Anschlußteil mit einer Austragleitung aufgesetzt wird.

Es kann vorgesehen sein, daß die Prüfleitung einen Leitungsabschnitt mit sehr engem Querschnitt und ein sich daran anschließendes Reaktionsvolumen mit vergrößertem Querschnitt aufweist. Der enge Leitungsabschnitt bildet dabei eine Prüfstrecke, an der ein großer Druckabfall auftritt und die vom Knochenzement dann nicht mehr passiert werden kann, wenn seine Viskosität zu hoch wird. Das Reaktionsvolumen mit vergrößertem Querschnitt dient als Auffangraum für den den engen Leitungsabschnitt passierenden Knochenzement und stellt sicher, daß auch bei etwas verzögertem Abschalten des Antriebes nach erfolgter Viskositätsprüfung kein unerwünschter Knochenzementaustritt erfolgt.

Die nachfolgende Beschreibung bevorzugter Ausführungsformen der Erfindung dient im Zusammenhang mit der Zeichnung der näheren Erläuterung. Es zeigen:
- Figur 1:: eine schematische Knochenzementaustragvorrichtung;
- Figur 2:: eine vergrößerte Teillängsschnittansicht des Vorratsbehälters der Vorrichtung der Figur 1 und einer daran angeschlossenen Prüfleitung;
- Figur 3:: eine Teilansicht des Vorratsbehälters der Vorrichtung der Figur 1 mit austauschbaren Anschlußteilen für eine Prüfleitung und für eine Austragleitung;
- Figur 4:: eine perspektivische Teilansicht des Vorratsbehälters der Vorrichtung der Figur 1 mit einem Anschlußteil mit Prüfleitung und Austragleitung;
- Figur 5:: eine Ansicht ähnlich Figur 3 mit einer abbrechbaren Prüfleitung und einer aufsetzbaren Austragleitung und
- Figur 6:: eine Ansicht ähnlich Figur 2 mit einer kombinierten Austrag- und Prüfleitung.

Die in der Zeichnung dargestellte Austragvorrichtung 1 für Knochenzement umfaßt einen zylinderförmigen Vorratsbehälter 2, in dem ein Kolben 3 abgedichtet verschiebbar ist. Der Kolben 3 ist über eine abgedichtete aus dem Vorratsbehälter 2 austretende Kolbenstange 4 und eine Kupplung 5 mit einem Antrieb 6 verbunden, der beispielsweise elektrisch, pneumatisch oder hydraulisch ausgebildet ist und der über die Kupplung 5 die Kolbenstange 4 und damit den Kolben 3 vorschiebt. Dadurch wird eine im Vorratsbehälter 2 enthaltene Füllung aus fließfähigem Knochenzement 11 durch eine Prüfleitung 7 hindurch gefördert, die am zentralen Stutzen 8 eines auf den Vorratsbehälter 2 aufgesetzten und diesen abschließenden Deckels 9 gehalten ist.

Die Prüfleitung 7 ist aus durchsichtigem Material hergestellt und trägt im Abstand von dem zentralen Stutzen 8 eine Markierung 10.

Der Knochenzement 11 im Inneren des Vorratsbehälters 2 wird üblicherweise durch Vermischung von zwei Komponenten angesetzt, durch die Vermischung beginnt eine Aushärtung mit Zunahme der Viskosität.

Bei geringer Viskosität gelangt bei Betätigung des Antriebs 6 und der dadurch erzeugten Verschiebung des Kolbens 3 Knochenzement 11 in die Prüfleitung 7 und schiebt sich an der Markierung 10 vorbei, der Benutzer kann dies erkennen und erhält somit einen Hinweis darauf, daß die Viskosität einen bestimmten Maximalwert noch nicht erreicht hat, der Knochenzement also für das Einbringen in einen Knochenhohlraum geeignet ist.

Steigt die Viskosität des Knochenzements 11 über einen bestimmten Maximalwert an, so erhöht sich dadurch auch die Kraft, die der Kolben 3 auf die Füllung des Knochenzements 11 aufbringen muß, um ein Eintreten des Knochenzements in die Prüfleitung 7 zu erreichen. Übersteigt diese Kraft einen bestimmten Wert, so unterbricht die Kupplung 5 die Antriebsverbindung zwischen dem Antrieb 6 und der Kolbenstange 4, das heißt der Kolben 3 im Vorratsbehälter 2 wird nicht mehr vorgeschoben und damit kann auch die Füllung des Knochenzements 11 nicht mehr in die Prüfleitung 7 eintreten. Der Benutzer kann dies daran erkennen, daß im Bereich der Markierung 10 kein Knochenzement in der Prüfleitung vorhanden ist, und damit erhält der Benutzer einen Hinweis darauf, daß die Füllung des Knochenzements 11 für das Einbringen in den Hohlraum nicht mehr geeignet ist, da die Viskosität einen Maximalwert überschritten hat.

Eine solche Prüfung wird regelmäßig vorgenommen werden, bevor mit dem Einfüllen des Knochenzements begonnen wird. Wenn der Einfüllvorgang begonnen worden ist, erfolgt das Einfüllen in der Regel zügig hintereinander, so daß während des sehr kurzzeitigen Einfüllvorgangs die Viskosität nicht so weit zunimmt, daß der Maximalwert der Viskosität überschritten wird.

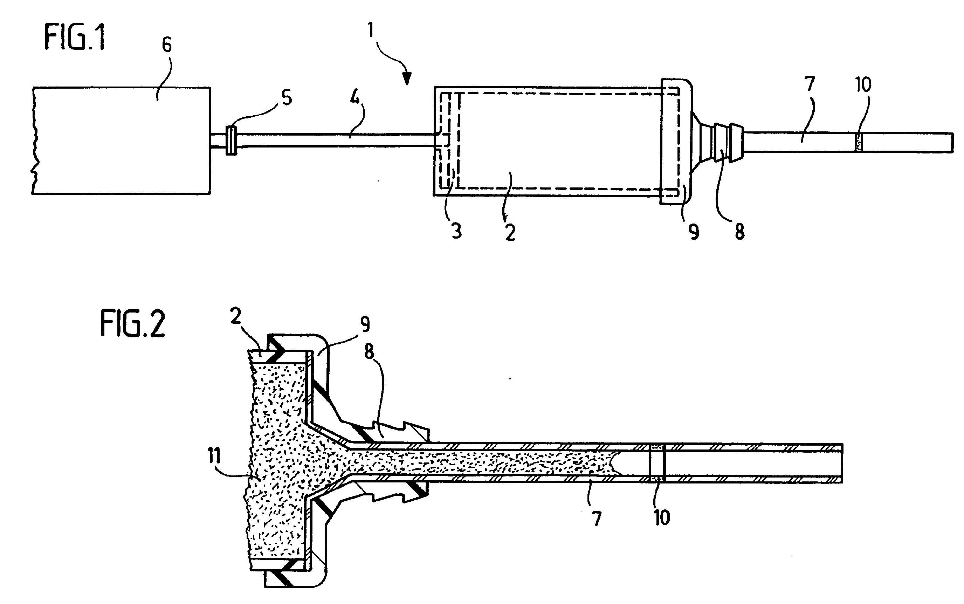

Bei dem in den Figuren 1 und 2 dargestellten Ausführungsbeispiel ist der Vorratsbehälter 2 lediglich mit einer Prüfleitung 7 verbunden, nicht jedoch mit einer speziellen Austragleitung zum Ausbringen des Knochenzements in den Knochenhohlraum.

Es ist möglich, daß die Prüfleitung 7 gleichzeitig die Austragleitung bildet, daß also der Benutzer die Prüfleitung 7 zum Ausbringen des Knochenzements unmittelbar auf eine Einfüllöffnung des Knochenzements an der Prothese aufsetzt.

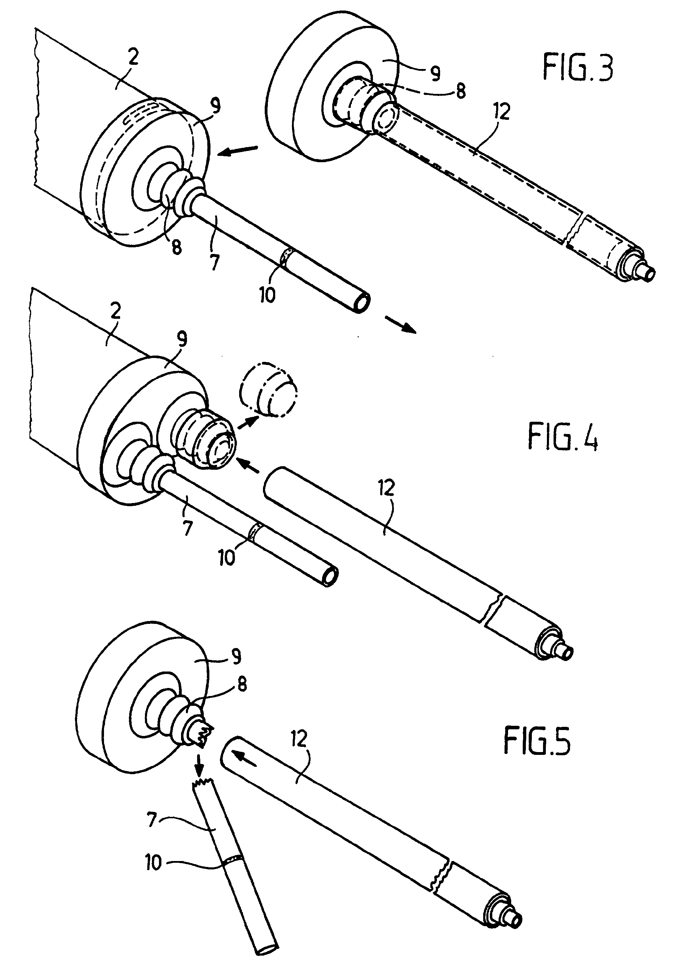

Bei einem anderen Ausführungsbeispiel ist vorgesehen, daß der Deckel 9 am Vorratsbehälter 2 austauschbar ist, wobei ein Deckel 9 mit einer Prüfleitung 7 verbunden ist, ein anderer Deckel 9 mit einer Austragleitung 12, die dann gegebenenfalls auch einen größeren Querschnitt aufweisen kann (Figur 3). Nach der Prüfung der Viskosität kann der Benutzer einfach durch Auswechseln der Deckel 9 mit den unterschiedlichen Leitungen den Vorratsbehälter 2 umrüsten zu einer Austragvorrichtung, so daß beim weiteren Vorschieben des Kolbens 3 Knochenzement über die Austragleitung 12 zu dem aufzufüllenden Knochenhohlraum gefördert wird.

Bei einem anderen Ausführungsbeispiel trägt der Deckel 9 nebeneinander sowohl eine Prüfleitung 7 als auch eine Austragleitung 12, wobei beide Leitungen gegebenenfalls verschließbar sind, beispielsweise durch eine statt der Prüfleitung 7 auf einen Anschlußstutzen aufgesetzte Verschlußkappe 13 (Figur 4). Ähnlich kann bei der Prüfleitung 7 vorgegangen werden, so daß beim Vorschieben des Kolbens 3 Knochenzement wahlweise in die Prüfleitung oder in die Austragleitung 12 gefördert wird.

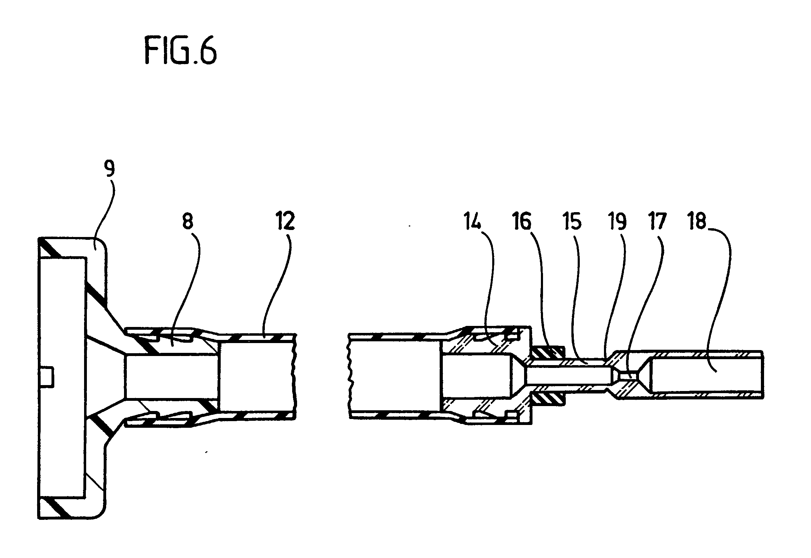

Beim Ausführungsbeispiel der Figur 5 schließlich wird die Prüfleitung 7 als abbrechbares Teil ausgebildet, daß nach Prüfung der Viskosität abgebrochen wird, statt der Prüfleitung 7 wird dann mit dem Deckel 9 die Austragleitung 12 verbunden, über die dann die Füllung des Knochenhohlraums erfolgt.

Bei dem Ausführungsbeispiel der Figur 6 ist auf den zentralen Stutzen 8 des Deckels 9 ein flexibler Schlauch aufgeschoben, der die Austragleitung 12 bildet, dieser Schlauch ist auf einen Adapter 14 aufgeschoben, der seinerseits einen Einfüllstutzen 15 und einen diesen umgebenden Dichtring 16 aufweist, dieser besteht beispielsweise aus Silikon.

Der Einfüllstutzen 15 geht über in die Prüfleitung 7, die ihrerseits einen gegenüber dem Einfüllstutzen 15 stark verengten Leitungsabschnitt 17 und ein sich stromabwärts daran anschließendes, im Querschnitt deutlich größeres Reaktionsvolumen 18 umfaßt.

Im Übergangsbereich vom Einfüllstutzen 15 zu dem Leitungsabschnitt 17 ist eine Bruchlinie 19 angeordnet, die ein Abbrechen des verengten Leitungsabschnittes 17 und des Reaktionsvolumens 18 vom Einfüllstutzen 15 ermöglicht.

Bei diesem Ausführungsbeispiel kann in der beschriebenen Weise zunächst die Viskosität des Knochenzementes geprüft werden, wenn die Viskosität unter dem zulässigen Maximalwert liegt, wird durch den Antrieb Knochenzement durch den engen Leitungsabschnitt 17 bis in das Reaktionsvolumen 18 gefördert, der Benutzer kann daran erkennen, daß der Knochenzement zur Verarbeitung geeignet ist. Daraufhin bricht der Benutzer längs der Bruchlinie 19 die Prüfleitung 7 ab und kann den Einfüllstutzen 15 sofort an die Einfüllöffnung des Implantates oder des aufzufüllenden Knochenhohlraumes dichtend ansetzen und mit dem Einfüllvorgang beginnen.

Im Bereich des Leitungsabschnittes 17, der einen sehr engen Querschnitt aufweist, ergibt sich ein großer Druckabfall, die Fördergeschwindigkeit des Knochenzementes ist jedoch aufgrund des engen Querschnittes relativ hoch. Im daran anschließenden Reaktionsvolumen 18, das einen wesentlich größeren Querschnitt aufweist, ergibt sich dagegen eine wesentlich geringere Fördergeschwindigkeit, so daß der Benutzer bei dem Test den Antrieb nicht sofort abschalten muß, wenn er sieht, daß der Knochenzement den engen Leitungsabschnitt 17 passiert hat, es bleibt ihm einige Zeit, bis das Reaktionsvolumen 18 mit Knochenzement aufgefüllt ist, ehe Knochenzement an der offenen Seite des Reaktionsvolumens 18 austritt. Auf diese Weise ist der Abschaltzeitpunkt nach dem Beenden der Viskositätsprüfung unkritisch, der Benutzer kann die Viskositätsprüfung ausführen, ohne daß unerwünscht Knochenzement aus der Vorrichtung austritt.

Die anhand der Figur 6 beschriebene Ausgestaltung der Prüfleitung 7 kann im übrigen auch bei den anderen Ausführungsbeispielen Verwendung finden.

## Patentansprüche

1. Verfahren zur Bestimmung der Viskosität eines fließfähigen Knochenzements, **dadurch gekennzeichnet, daß** man den fließfähigen Knochenzement (11) mit einer Austragvorrichtung aus einem Vorratsbehälter (2) durch eine Prüfleitung (7) mit einer begrenzten Austragskraft hindurchfördert und beobachtet, wie weit der Knochenzement (11) aus dem Vorratsbehälter (2) in die Prüfleitung (7) gedrückt wird.

2. Verfahren nach Anspruch 1, **dadurch gekennzeichnet, daß** man den Knochenzement (11) bis zum Erreichen einer maximalen Austragskraft mit konstantem Volumenstrom in die Prüfleitung (7) fördert.

3. Vorrichtung zur Bestimmung der Viskosität eines fließfähigen Knochenzements mit einem Vorratsbehälter (2) für den fließfähigen Knochenzement (11), einer aus diesem austretenden Austragleitung und einer Fördereinrichtung (3, 6), die den fließfähigen Knochenzement (11) aus dem Vorratsbehälter (2) in die Austragleitung fördert, **dadurch gekennzeichnet, daß** der Vorratsbehälter (2) mit einer Prüfleitung (7) verbunden ist, daß der Fördereinrichtung (3, 6) ein Schaltelement (5) zugeordnet ist, welches die Fördereinrichtung abschaltet, sobald ein Maximalwert einer Förderkraft überschritten wird und daß die Prüfleitung (7) derart beschaffen ist, daß feststellbar ist, wie weit Knochenzement (11) in die Prüfleitung (7) eingetreten ist.

4. Vorrichtung nach Anspruch 3, **dadurch gekennzeichnet, daß** das Schaltelement eine Kupplung (5) ist.

5. Vorrichtung nach Anspruch 3 oder 4, **dadurch gekennzeichnet, daß** die Prüfleitung (7) durchsichtig ist.

6. Vorrichtung nach Anspruch 5, **dadurch gekennzeichnet, daß** die Prüfleitung (7) im Abstand zum Vorratsbehälter (2) eine Markierung (10) trägt.

7. Vorrichtung nach einem der Ansprüche 4 bis 6, **dadurch gekennzeichnet, daß** die Prüfleitung (7) durch die Austragleitung gebildet wird.

8. Vorrichtung nach einem der Ansprüche 4 bis 6, **dadurch gekennzeichnet, daß** aus dem Vorratsbehälter (2) eine Prüfleitung (7) und eine Austragleitung (12) austreten.

9. Vorrichtung nach Anspruch 8, **dadurch gekennzeichnet, daß** die Austragleitung (12) und die Prüfleitung (7) verschließbar sind.

10. Vorrichtung nach einem der Ansprüche 4 bis 9, **dadurch gekennzeichnet, daß** die Prüfleitung (7) abbrechbar ausgebildet ist.

11. Vorrichtung nach Anspruch 10, **dadurch gekennzeichnet, daß** sich die Prüfleitung (7) unmittelbar an die Austragleitung (12) anschließt.

12. Vorrichtung nach einem der Ansprüche 4 bis 6, **dadurch gekennzeichnet, daß** am Vorratsbehälter (2) austauschbare Anschlußteile (9) befestigbar sind, die wahlweise mit der Austragleitung (12) oder mit einer Prüfleitung (7) verbunden sind.

13. Vorrichtung nach einem der Ansprüche 3 bis 12, **dadurch gekennzeichnet, daß** die Prüfleitung (7) einen Leitungsabschnitt (17) mit engem Querschnitt umfaßt, an den sich ein Reaktionsvolumen (18) mit vergrößertem Querschnitt anschließt.

## Claims

1. A method of determining the viscosity of a flowable bone cement, **characterised in that** the flowable bone cement (11) is conveyed, by means of a discharging device and at a limited discharging force, from a storage vessel (2) through a test line (7) and it is observed how far the bone cement (11) is pushed from the storage vessel (2) into the test line (7).

2. A method according to claim 1, **characterised in that** the bone cement (11) is conveyed into the test line (7) at a constant volume flow until a maximum discharging force is achieved.

3. A device for determining the viscosity of a flowable bone cement, having a storage vessel (2) for the flowable bone cement (11), a discharge line issuing therefrom and a conveying device (3, 6) conveying the flowable bone cement (11) from the storage vessel (2) into the discharge line, **characterised in that** the storage vessel (2) is connected to a test line (7), **in that** a control element (5) is associated with the conveying device (3, 6), which control element (5) deactivates the conveying device as soon as a maximum value of a conveying force is exceeded, and **in that** the test line (7) is such that it can be ascertained how far bone cement (11) has entered the test line (7).

4. A device according to claim 3, **characterised in that** the control element is a coupling (5).

5. A device according to claim 3 or 4, **characterised in that** the test line (7) is transparent.

6. A device according to claim 5, **characterised in that** the test line (7) bears a marking (10) at a distance from the storage vessel (2).

7. A device according to any one of claims 4 to 6, **characterised in that** the test line (7) is formed by the discharge line.

8. A device according to any one of claims 4 to 6, **characterised in that** a test line (7) and a discharge line (12) issue from the storage vessel (2).

9. A device according to claim 8, **characterised in that** the discharge line (12) and the test line (7) can be closed.

10. A device according to any one of claims 4 to 9, **characterised in that** the test line (7) can be broken off.

11. A device according to claim 10, **characterised in that** the test line (7) is directly attached to the discharge line (12).

12. A device according to any one of claims 4 to 6, **characterised in that** exchangeable connecting parts (9) can be secured to the storage vessel (2) and are optionally connected to the discharge line (12) or to a test line (7).

13. A device according to any one of claims 3 to 12, **characterised in that** the test line (7) comprises a line portion (17) of narrow cross-section, to which a reaction volume (18) of enlarged cross-section is attached.

## Revendications

1. Procédé de détermination de la viscosité d'un ciment pour os coulant, **caractérisé en ce que** l'on fait passer le ciment pour os coulant (11) avec un dispositif d'extraction à partir d'un réservoir de stockage (2) à travers une ligne d'essai (7) avec une force d'extraction limitée et on observe dans quelle mesure le ciment pour os (11) est comprimé du réservoir de stockage (2) à la ligne d'essai (7).

2. Procédé selon la revendication 1, **caractérisé en ce qu'**on refoule le ciment pour os (11) dans la ligne d'essai (7) jusqu'à ce que l'on atteigne une force d'extraction maximale avec un débit volumique constant.

3. Dispositif de détermination de la viscosité d'un ciment pour os coulant comportant un réservoir de stockage (2) pour le ciment pour os coulant (11), une conduite d'extraction sortant de celui-ci et un dispositif de refoulement (3, 6) qui refoule le ciment pour os coulant (11) du réservoir de stockage (2) à la conduite d'extraction, **caractérisé en ce que** le réservoir de stockage (2) est raccordé à une ligne d'essai (7), **en ce qu'**au dispositif de refoulement (3, 6) est attribué un élément de commutation (5), lequel arrête le dispositif de refoulement dès qu'une valeur maximale d'une force de refoulement est dépassée et **en ce que** la ligne d'essai (7) est réalisée de telle sorte que l'on puisse déterminer dans quelle mesure le ciment pour os (11) a pénétré dans la ligne d'essai (7).

4. Dispositif selon la revendication 3, **caractérisé en ce que** l'élément de commutation est un accouplement (5).

5. Dispositif selon la revendication 3 ou 4, **caractérisé en ce que** la ligne d'essai (7) est transparente.

6. Dispositif selon la revendication 5, **caractérisé en ce que** la ligne de vérification (7) comporte un marquage (10) à distance du réservoir de stockage (2).

7. Dispositif selon l'une quelconque des revendications 4 à 6, **caractérisé en ce que** la ligne d'essai (7) est formée de la conduite d'extraction.

8. Dispositif selon l'une quelconque des revendications 4 à 6, **caractérisé en ce qu'**une ligne d'essai (7) et une conduite d'extraction (12) sortent du réservoir de stockage (2).

9. Dispositif selon la revendication 8, **caractérisé en ce que** la conduite d'extraction (12) et la ligne d'essai (7) peuvent être obturées.

10. Dispositif selon l'une quelconque des revendications 4 à 9, **caractérisé en ce que** la ligne d'essai (7) est réalisée de manière à pouvoir être rompue.

11. Dispositif selon la revendication 10, **caractérisé en ce que** la ligne d'essai (7) se raccorde directement à la conduite d'extraction (12).

12. Dispositif selon l'une quelconque des revendications 4 à 6, **caractérisé en que**, sur le réservoir de stockage (2), il est possible de fixer des pièces de raccordement échangeables (9) qui sont raccordées au choix à la conduite d'extraction (12) ou à une ligne d'essai (7).

13. Dispositif selon l'une quelconque des revendications 3 à 12, **caractérisé en ce que** la ligne d'essai (7) comporte une section de conduite (17) avec une section transversale étroite à laquelle se raccorde un volume de réaction (18) avec une section transversale agrandie.
